# EUROPEAN PATENT SPECIFICATION

(11) **EP 0 789 778 B1**
(45) Date of publication and mention of the grant of the patent: **30.05.2001**
(21) Application number: 95910682.4
(22) Date of filing: 13.03.1995
(51) Int. Cl.: C12Q 1/04, C12Q 1/06, F04B 43/14, C12M 1/00, C12M 1/34

(54) **CELLULAR MATERIAL DETECTION APPARATUS AND METHOD**
ZELLMATERIALNACHWEISGERÄT UND VERFAHREN
DISPOSITIF ET PROCEDE DE DETECTION DE MATERIAU CELLULAIRE

(30) Priority: 18.03.1994 GB 9405392
(43) Date of publication of application: 20.08.1997
(73) Proprietor: SECRETARY OF STATE FOR DEFENCE IN HER BRITANNIC MAJESTY'S GOV. OF THE UNITED KINGDOM OF GREAT BRITAIN AND NORTHERN IRELAND, London SW1A 2HB (GB)
(72) Inventor: SQUIRRELL, David, James CBDE Porton Down, Wiltshire SP4 0JQ (GB)
(74) Representative: Skelton, Stephen Richard
(86) International application number: PCT/GB95/00544
(87) International publication number: WO 95/25811

(56) References cited:
- CH-A- 653 414
- JP-A- 3 112 495
- JP-A- 5 184 350
- JP-A- 60 016 598
- PATENT ABSTRACTS OF JAPAN vol. 11 no. 303 (P-622) [2750] ,3 October 1987 & JP,A,62 093634 (MATSUSHITA SEIKO COMPANY) 30 April 1987, cited in the application

## Description

The present invention relates to a method and apparatus for monitoring a gaseous environment for the presence of cellular material; more particularly it relates to an apparatus that is capable of providing a measure of presence and/or numbers of cellular microorganisms, such as bacterial cells, in a large volume of air such as in a warehouse or production facility or in an open air location where bacterial presence is suspected. The method and apparatus of the invention are particularly provided for determining the likelihood of pathogenic or allergenic material being present in an environment by continuous on-line measurement of cell numbers. The latter format provides a continuous monitoring of an environment.

There is a military need for detection of the incidence of attack using biological materials, including attack using bacteria eg. in the form of cells or spores. Such need includes the capability to monitor the air some distance upwind of an asset site in order that sufficient warning may be given to personnel on that site that an attack with bacteria is imminent. In such circumstances it is required that monitoring be carried out continuously, that is for a continuous period of time for any one monitoring device eg. from several to several tens of hours.

There is a further need for determining the presence of pathogens in facilities such as hospitals and in manufacturing premises in which foodstuffs, sterile pharmaceuticals or physiological supplements are being placed in containers prior to use. Before a production run it is desirable that the sterility of the packing environment be checked for the presence of pathogens or less harmful bacteria that may be used as an indication of likely presence of pathogens or allergens.

In both of these situations it is necessary to process a large volume of air, either because of the continuous nature of the measurement or because of the need to sample a significant amount of clean room or sterile warehouse air. Furthermore in both situations it is necessary to screen for a wide range of bacteria, regardless of type, as the threat may not be that of a known genus or species.

It is known to use the luminol reaction to analyse air for the presence of haematin, but this technology is susceptible to giving readings with inorganic materials and is limited to a detection limit of 103 bacterial cells in theory as only 10⁻¹⁶ grams of haematin is extractable from the average bacterial cell. Metal sensitivity giving high backgrounds render this system unreliable in practice.

It is known to screen for the likely presence of bacteria by analysing samples for the presence of adenosine triphosphate. This is readily carried out using luciferase and luciferin agent whereupon the presence of ATP allows luciferase to catalyse the oxidation of luciferin with the resultant emission of light. Samples are loaded into a luminometer and the amount of light emitted used as a measure of the amount of bacteria present. In order to liberate as much ATP from any cells present it is known to add a detergent to the sample in order to lyse the cells and release the ATP.

Although such biochemistry has been extensively utilised with individual samples derived by direct sampling of surfaces, liquids and solids, there has been little development of luminometry equipment suitable for monitoring bacteria in air.

JP 62093634 discloses a counter for microorganisms which draws in an air sample, collects the microorganisms from that and extracts ATP from them before assaying the ATP using a luminescent reaction. This device uses a 0.2µ membrane filter to collect microorganisms from the air in a batchwise fashion, with the membrane being periodically analysed by being passed to an extracted station. No details of the sensitivity of this equipment are given, but its performance is limited by the ability of the air pump to draw sufficient sample air across the membrane and by the time taken to process the membrane microorganism content.

JP 60016598 discloses a method for detecting bacteria in air again using luminescent reagents to assay ATP. This method extracts ATP using a Tris-EDTA liquid buffer heated to 100°C from samples of 10s of litres of air per minute batched in 10 minute samples. Filters are required to eliminate dirt and dust and these are described as essential to the method. A cooling tube is required in order to avoid increase of background noise due to raising of temperature of photomultiplier tubes use to monitor luminescence. This apparatus also uses an 'extractor' to draw air into to at 10s of litres per minute. The exact nature of this 'extractor' is not clear.

It is known to use cyclone devices to capture particulates from air, these devices typically being electrically driven and producing particulate depleted and particulate concentrated fractions. It is known to use such devices for the purposes of obtaining aerosols and other particulates from air for later analysis. For example GB 2245024 describes a cyclone for collecting a large sample volume of biological materials from the air; SU 1546481 and SU 11911460 describe use of cyclones to provide a particulate sample which is used to seed nutrient holding vessels or plates for analysis while SU 916535 collects bacteria from such cyclone on a filter band and viruses in a lower section wherefrom they are used to infect experimental animals. It is also known to use virtual impactors to collect airborne particulates, see eg. US 4942297 and US 4670135.

Again, none of these systems are capable of continuous monitoring of air for the presence of bacteria, particularly small amounts of pathogenic bacteria. A particular problem is the variation in concentration of fluid output from the cyclone with changes in humidity of the air being sampled. With very high throughput the cylone can run almost dry and produce high readings from a relatively normal background input.

JP 5184350 describes a system for counting of bacterial cells suspended in air which aims to shorten the determination time and improve the accuracy of the results. JP 60016598 describes an alternative device for detecting , bacteria in an amount of air. JP 3112495 describes a filter system for the detection of different microorganisms floating in air. None of these devices are suitable for continuous operation.

The present inventor has now provided a truly continuous flow luminometry method and apparatus that are capable of continuous or batch monitoring of bacteria in a gaseous environment, particularly atmospheric air, such that on-line measurements may be taken of air bacteria content. Such apparatus is particularly directed at continuous monitoring but is equally suitable for sampling large volumes of air such as those inside a hospital, manufacturing facility clean room or sterile warehouse for sampling before a production run.

In a first aspect the present invention provides a method for determining the presence and/or amount of cellular material present in a gaseous environment comprising:
(a) continuously collecting a particulate fraction from that environment over a period of time;
(b) continuously transferring the particulate fraction to a stream of processing fluid;
(c) continuously releasing intracellular contents including ATP from microorganisms cells or spores present in the said stream of processing fluid containing the particulate fraction;
(d) continuously adding luminescent reagents dependent upon presence of ATP to the stream of processing fluid to effect luminescence;
(e) measuring light emitted from the stream of processing fluid produced in (d) in a luminometer wherein a signal indicative of this light is produced by the luminometer and the presence and magnitude of the signal is equated to presence and/or amount of cellular material present in the gas.

Preferably the gas is atmospheric air, the material bacteria, and steps (b), (c), (d) and/or (e) are carried out continuously. Preferably step (b) is carried out using a detergent, but it may be carried out by use of heat, sound or other energy source or a lytic agent, eg an enzyme, with suitable cooling effect applied if excess heat has been generated.

In order to carry out all these steps continuously it is preferred to pass the processing fluid from the collecting step to the downstream steps using a conduit, whereby the time taken for bacteria collected in step (a) to be identified as such by their ATP content is limited only by the time taken for the fluid to pass down the conduit to the luminometry steps.

More preferably the passage of fluid down the conduit is controlled by a drive means such as one or more pumps whereby a constant flow of collecting fluid may be analysed by luminometry.

For the purpose of collecting the particulate sample it is preferred to suck air into a collecting device, deposit cellular material, eg. bacteria in the form of particulates into a collecting part of the collector device and discharge particulate depleted air from the collector. Such collection is preferably carried out at a rate of some tens to thousands of litres of air per minute in order that a useful sample is taken; conveniently at 100-1000 litres per minute.

In order to differentiate between bacteria and other cellular materials such as eucaryotic cells, eg. pollens, or fungal spores it is possible to split the particulate containing processing fluid into two streams, or use two collectors to produce two processing fluid flows, and add a detergent capable of releasing cell content including ATP from all cells and spores to one fluid flow and one that is only capable of releasing the content of one of the eucaryotic cells and fungal spores to the other. For such purpose it is possible to add eg. non-ionic detergent for releasing materials from eucaryotic cells and fungal spores and eg. cationic detergent for releasing it from all cells.

By subtracting the signal from the non-ionic detergent flow luminometer from that from the cationic detergent flow luminometer it is possible to produce a continuous signal indicative of bacterial presence and numbers.

In a still more preferred method of the invention bacteria are detected by detection of the amount of adenylate kinase activity in the processing fluid containing the collected particulates, wherein adenosine diphosphate (ADP) is added to the processing fluid and is converted by any adenylate kinase present to adenosine triphosphate which in turn is detected as described above. In this manner the sensitivity of the method is increased due to the cascade effect of the enzyme's activity leading to effective amplification of signal. Such a method when applied to bacterial detection in general is the subject of the applicant's copending application PCT/GB94/00118 by the same inventor. ADP may be included in the processing fluid as it enters the particulate fraction collection step, or may be added downstream eg. with any reagents added before the luminescent reagents.

The purity of the ADP added should preferably be such that the ADP to ATP ratio in the reagent is 2000:1 or more; more preferably 6000:1 or more. The concentration of ADP in the processing fluid may in theory be any level where it is in excess of the ATP already present in the organisms if significant sensitization is to be produced. It is preferred to use at least 0.01 mM or more ADP, more preferably 0.01 to 1mM ADP or more as the final concentration in the processing fluid.

While cellular material contains sufficient magnesium for the magnesium dependent conversion of ADP to ATP, it will be realised by those skilled in the art that the ADP reagent should be preferably be used in the presence of magnesium ions if ATP is to be optimally produced. Thus the ADP reagent is preferably made up in a buffer containing sufficient magnesium to provide magnesium levels at least as two or more times the molarity as the ADP. Preferably the conversion of ADP to ATP takes place in a buffer solution of pH5.5 - 8.5 buffer, more preferably pH7.8. The provision of magnesium is particularly preferred where ADP is stabilised using EDTA or like chelating agents.

It will be realised by those skilled in the art that the term continuously as applied to step (a) herein is intended to cover the collection of particulates continuously over an operating period.

Such period may be from several minutes to many hours or may be a set period of time typically prior to operation of a sterile processing line. For use in an interior sterile space this period will be sufficient for the apparatus to have collected particulates from a volume equal to a significant proportion the air of that space, potentially substantially all of it, and may be collected in batches.

In a preferred form of the method wherein a sterile space such as a hospital building, clean-room or sterile packing installation is being monitored the method continuously collects particulates from the air while various vents supplying air conditioned air are operated or closed down. Processing fluid corresponding to the air collected during operation of each air vent and processing fluid collected with the conditioning system off are monitored using the on-line ATP measuring capability of the method of the invention, whereby a rapid indication of the location of any contamination is provided if any of the sets of fluid show increased bacterial presence. Where collection and processing fluid production is running for the entire period of test, as opposed to just collecting batches of particulates, it is possible to merely correlate the output of the luminometer at a particular time to the presence of bacteria.

In a second aspect of the present invention there is provided an apparatus suitable for carrying out the method of the present invention.

The apparatus of the second invention comprises:
(a) a means for continuously collecting a particulate fraction from a gaseous environment;
(b) a means for continuously transferring the particulate fraction to a stream of a processing fluid;
(c) a means for continuously releasing intracellular contents including ATP from cellular material present in the stream of processing fluid;
(d) a means for continuously adding luminescent reagents, dependent upon presence of ATP to effect luminescence, to the stream of processing fluid;
(e) a light detector means adapted to be fed with the processing fluid from step (d) and capable of emitting a signal indicative of the occurrence and amount of luminescence detected thereby; and
(g) a signal transmitting means for feeding the signal from the luminometer to a processor and/or display means for indicating the presence and/or amount of microorganism cells or spores.

The continuous collecting means (b) is conveniently provided in the form of a cyclone or a virtual impactor, preferably a high volume virtual impactor. Where continuous monitoring of external air, i.e. outside buildings, is required it is preferred to utilise a cyclone, preferably one capable of processing between 100 and 1000 litres or more of air per minute and providing a particulate fraction therefrom on a continuous basis; no upper limit is necessary however as much higher capacity cyclones will be known to those skilled in the art. Preferably the cyclone is a wet walled hydrocyclone and the particulate fraction is provided in the form of a liquid processing fluid containing particulate as it leaves the cyclone. Where a limited volume of gas is to be sample, such as in a sterile production line unit, it may be preferred to use a high velocity virtual impactor to remove particulates from the air; such impactor might conveniently be capable of sampling about 50 to 150 litres of air per minute.

With both of these options the means for transferring the particulate fraction to processing fluid preferably comprises a supply of processing fluid into which the fraction is deposited. The processing fluid is preferably a liquid such as water or a buffer, optionally containing magnesium ions, ADP and/or reagent for releasing intracellular contents including ATP from cells.

Where large volumes of gas are being sampled with variation in humidity it is preferred to add any detergent downstream of the collector and use a gas liquid interface that is capable of maintaining the dilution of the particulate in the processing fluid at a substantially constant level and remove excess air as bubbles. One suitable gas liquid interface is that described in copending application EP-A-668095.

A preferred modification of this interface receives liquid processing fluid under influence of a pump between the collector and interface. When the liquid level in the interface falls below a set level a level sensor transmits a signal to the pump to cause it to increase fluid flow rate. At the same time air entrained as bubbles in the liquid is allowed to escape to the atmosphere thus ensuring a reasonably constant supply of liquid to the downstream stages of the apparatus.

A further modification of this device places the pump downstream of the interface, and the pump is slowed when the interface liquid level falls below the set level such that the liquid level might recover. The flow rate of processing fluid through the cyclone may be any flow to which a suitable amount of detergent, ADP, phosphate and luminescence reagents can be supplied without logistical problems. A convenient flow rate is from between 0.1 and 10 ml processing fluid per minute where 1000 litres of air minute is entering the collector, e.g. cyclone, at the same time.

The means for releasing intracellular content including ATP may include a heater, sonic device or device for adding a lytic agent such as an enzyme or detergent as described above in the description of the method. Where the means relies on physical effect to release cell content it must produce that effect at or downstream of the means for transferring particulates to the processing liquid. Where the means adds a lytic agent it may be positioned upstream, at or downstream of the transferring means. A cooling device may be included before the downstream luminescence steps if heating is used.

The processing fluid is passed from its supply, ie. a reservoir, through the particulate transferring means and to any downstream intracellular contents releasing means by means of a fluid flow path. Preferably this is provided in the form of a liquid conduit, more preferably in the form of tubing. The processing fluid is preferably liquid moved through the conduit by means of one or more pumps and preferably these are peristaltic pumps which act upon the liquid through the conduit wall; preferred conduits being of flexible tubing type, more preferably flexible transparent plastics tubing suitable for delivering liquid under the influence of peristaltic pumps.

Using flow rates of from 0.1 to 10 ml processing fluid per minute to the cyclone, it may be expected that evaporation will reduce flow still further such that for example 0.05 to 10ml per minute, but preferably 0.5 to 5ml per minute, will enter the interface. At such flow rates the apparatus can conveniently use peristaltic tubing of the order of 0.25 to 3mm internal diameter, although no particular limit is placed here other than the fact that the tubing should not be too wide to retain an air free flow through the apparatus. Suitable silicone rubber tubing for this purpose is available from Autoclude at 0.8mm internal diameter; alternative tubing is available from Watson Marlow-UK or Ismatec-Switzerland.

Where a lytic agent is added downstream of the particulate transferring means a junction is preferably provided whereby a single peristaltic pump acts upon one or more tubes carrying processing fluid from the transferring means and one or more tubes carrying the lytic agent, preferably a solution of lytic agent; at least one of each type of tube feeding into a single downstream tube at a junction between the two.

A similar arrangement, preferably with a separate peristaltic pump, is provided for the optional addition of any ADP reagent in the adenylate kinase based aspect of the method whereby enhanced amounts of ATP are derived from a given number of bacteria and similarly for the means for adding luminescent reagents. In the latter case the junction between the conduit carrying processing fluid from step (c), with the conduit carrying luminescence reagents is preferably effected in the light measuring device itself, eg. a luminometer light measuring chamber.

Where the liquid processing fluid plus particulates is being divided into two flows for processing with different lytic agents it is possible for these to be produced by separate cyclones or impactors, at the processing fluid outlet of a single cyclone or impactor, at the air liquid interface or downstream of these. One embodiment of the apparatus of the invention provides these streams by use of a manifold downstream of the interface, and preferably uses this manifold for mixing the two lytic agents with respective ones of these streams. Thus the manifold has typically a central inlet from the interface carrying for example approximately 50% of the liquid flow input, and preferably 80%, to the collector (dependent upon evaporation in the cyclone or impactor) and that is flanked by respective inlets from supplies of the two detergent reagents used at eg. 25% of that central inlet flow each. Two outlets from the manifold provide combined flows made up of 50:50 mixes of the central flow and the respective detergent flow. Typically this might be of the order of 75% to 125% of volume of the flow from the interface from each of the manifold outlets.

In a preferred embodiment of the apparatus of the invention, particularly suited to outdoor monitoring such as would be required in protecting a military asset, it is preferred to have two flows of processing fluid and treat these with different intracellular contents releasing means in order to distinguish between eucaryotic cells and fungal spores on one hand, and bacteria on the other. These flows may emanate from a single particulate collector or from two separate collectors operated at the same location. The preferred detergents used for such flows are those as described above for the method of the invention and as disclosed in PCT/GB94/00118.

In an alternative embodiment of the invention one of the luminescent agents, preferably luciferase, is immobilised near the luminometer light detector within the light measuring chamber where the processing fluid and luminescence reagents, in this case containing luciferin solution, are mixed. By being immobilised near the detector the efficiency of the detection of light may be maximised. In order to maintain the activity of the luciferase this may be fixed on a ribbon like substrate that is wound from one reel to another such that fresh luciferase is presented to the incoming fluids at a desired rate. Immobilisation may be achieved by chemically or physically linking the enzyme, eg. using a chemical linker group such as glutaraldehyde, to a suitably derivatised planar substrate, and the liquid tight nature of the chamber may be retained by introducing the ribbon of this substrate through eg. rollers that are liquid tight or are provided above the liquid level.

It will be realised that the method and apparatus of the invention may operate at ambient temperatures, but may also be operated at higher temperatures where reagent heat stability so permits. Thus the tubing or the luminometer light measuring chamber may be heated in order to increase the amount ATP produced by the ADP agent or the light emitted from the luminescent reagents in response to presence of ATP. For such use the thermostable luciferases of the applicant's copending PCT/WO95/25798 (which superseded British Patent Application Nos. 940570.2, 9501170.6 and 9500660.7) may be used.

It will be realised by those skilled in the art that after exiting the luminometer chambers, e.g. by further peristaltic tubing, the flows may be forwarded to further analytical devices such as those using specific binding to more specifically determine the nature of any bacteria present, or other agents such as viruses, DNA or chemical agents.

Further provided by the method of the invention is a tubing element or network of such elements suitable for use in the apparatus of the invention comprising a peristaltic tubing element or network of such elements wherein the element or network has a first tubing length with a free end which is suitable for attachment to the fluid outlet of an apparatus for continuously collecting a particulate fraction from a gaseous environment, a further tubing length which has a free end which is suitable for connection to an inlet means of a luminometer chamber, other ends of said first and further tubing lengths being interconnected by a tubing length which includes two or more junctions, each junction being connected to a reagent container by means of a tubing length, all the lengths being capable of peristaltic action under influence of a peristaltic pump, characterised in that each reagent container contains a reagent for use in the method described above, the amount and concentration of said reagents being such that the tubing network may be included within an apparatus described above and the various reagents in the containers will last similar lengths of operation time when the apparatus is used for performing the method.

The element or network of the invention may comprises yet more junctions between the first and second tubing lengths with lengths of tubing having free ends suitable for connection to further sources of reagents.

Thus where detergent is used to lyse cellular material and is added upstream of the particulate collector, and bacterial ATP is being measured directly, a tubing element merely joining the interface and luminometer light measuring chamber may be used. Where the detergent is added downstream of the collector and the adenylate kinase variant of the method is being employed two junctions will be required to allow linkage with lengths of tubing from a detergent and ADP reagent source respectively. A separate luminescent reagent source and tubing may be included with the element or network as part of a reagent replenishment kit.

In each case it is preferred, by the very nature of the apparatus, that the free ends of the tubing, including those leading to reagent bottles, be covered to exclude the entry of bacteria and other cellular materials, preferably by a puncturable or removable end cover, eg. removable by tearing, cutting or pulling. The free ends to the reagents are actually attached to the reagents required such that a sterile replacement tubing network might be replaced all in one go with the amounts and concentrations of the various reagents being matched to each other such that they last similar lengths of operation time.

The method, apparatus and tubing element and networks of the present invention will now be exemplified by way of illustration only by reference to the following non-limiting Examples and Figures. Further embodiments of the invention falling within the scope of the claims will occur to those skilled in the art in the light of these.

### FIGURES:

Figure 1: shows a diagrammatic representation of an apparatus of the invention comprising a cyclone and gas-liquid interface feeding twin lines with non-ionic and cationic detergent feeds, luminescence reagent feeds, luminometers and a central processing unit.

Figure 2: shows a cross section through a cyclone and gas liquid interface as used in the apparatus of Figure 1.

Figure 3; shows a tubing element and reagent container network suitable for replacement of reagents as one unit for an apparatus as described in Example 2.

Figure 4: is a graph of a counts per minute output of a luminometer as described in Example 1 when various amounts of ATP are added into the tubing directly downstream of the air/liquid interface.

### EXAMPLES

### EXAMPLE 1: Continuous flow luminometer apparatus.

A continuous flow luminometer apparatus of the invention was constructed using a cyclone unit (1), capable of removing particulates from approximately 1000 litres of air per minute using water as the processing fluid, connected to a gas liquid interface device (2) downstream for degassing the fluid and splitting the flow into two parallel processing flows. In this manner the liquid collects the particulates, including any aerosols, and carries them under influence of action of peristaltic pumps (4) via peristaltic tubing conduits (3) of 0.8mm silicone rubber (Autoclude) to junctions (5) where the influence of pumps (5) draws a flow of detergent (either 0.2% aqueous CTAB solution or 0.4% aqueous Triton X-100) from containers (6) into the processing liquid flow (giving a flow concentration of 0.1% CTAB or 0.2% Triton X-100). Further pumps (7) draw the fluid flows on and deliver them at the same rate as a solution containing a flash kinetic mixture of luciferase, luciferin and buffer (Biotrace plc Bridgend, UK) from containers (8) to a light measurement chamber (not shown) within a luminometer housing (9) where the respective flows and reagents are mixed. The pumps (7) achieve synchronous delivery by acting upon delivery lines (10) and (11) simultaneously and the relative amounts of ATP released by the same particulate sample under influence of the detergents provides amounts of light and thus signals indicative of total cells/spores and eucaryotic cells/spores.

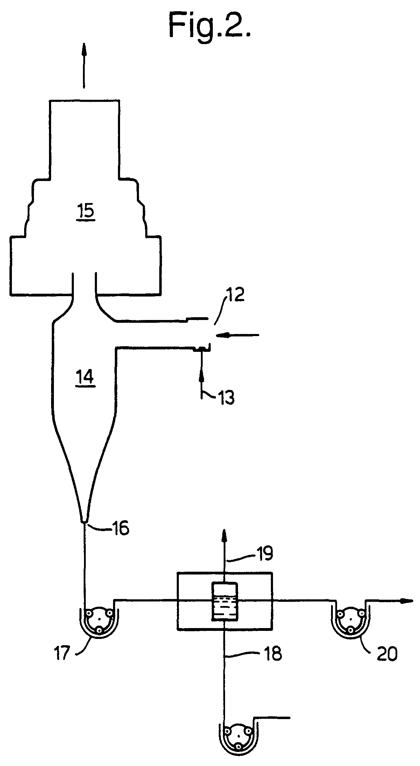

The cyclone is shown in more detail in Figure 2, where (12) is an inlet for gas (atmospheric air) to be separated into particulate enriched and particulate depleted fractions, (13) is a supply of water processing liquid pumped at 1 ml/minute which is entrained with the air flow and passes therewith into the cyclone body main volume (14) where particulate depleted air exits under influence of an air mover (15) while processing fluid and entrapped particulates pass downward to an outlet (16) in the bottom of the cyclone. A peristaltic pump (17) passes the processing fluid to a gas liquid interface of capacity approx. 100µl where bubbles are removed; the rate of supply of the processing fluid by the pump, or alternatively the removal of fluid by a pump downstream, being determined by the liquid level in the interface. Liquid level is determined by a liquid level sensor (not shown) and where the level falls below a set height the pump is operated to draw more processing liquid through until the desired level is restored. Excess liquid and dense particulates from the interface are removed periodically from outlet (18), air leaving the liquid as bubbles is removed via outlet (19) and processing liquid is passed under influence of a peristaltic pump (20) to the downstream tubing for addition of reagents.

As the processing liquid with particulates and detergent mixes with the luminescent reagents in the luminometer light measurement chamber, a light sensor (not shown) measures any light emitted in the chamber resultant from the presence of ATP; emission being rapid due to the flash kinetic ratio reagent having excess luciferase compared to luciferin over the ratio required for glow kinetics (where measurement is over several minutes). The luminometer transmits a signal representative of light emitted to a computer processor which in turn may display this on a display unit or compare it with control levels.

The detergents used may be standard chemicals as described above but may be provided in the form of specialist proprietary extractants such as Enzymatics ATP releasing agent, Biotrace XM extractant (Biotrace, Bridgend, UK) or Lumac NRM (Lumac BV Holland). The luminescence reagents may be standard reagents available from suppliers such as Biotrace plc and Celcis plc UK and others; the luciferase may be natural or recombinant. Concentrations of reagents are those necessary to produce flash kinetics and their ratio to the amount of cetyl trimethyl ammonium bromide treated processing liquid is that which the manufacturers recommend for batch processing taking into account the relative sizes of the tubing from each line entering the luminometer chamber. The speed of peristaltic pumps may be also adjusted to maintain ratios where separate pumps supply each flow.

### EXAMPLE 2: Adenylate kinase measuring continuous flow luminometer.

A second embodiment of the luminometer of the present invention is provided wherein an additional reagent flow is included for feeding very pure adenosine diphosphate (ADP) reagent, with respect to ATP, into the processing fluid downstream of the junction (5) where detergent is added, but upstream of the luminometer. The ADP reagent (99.95% pure with respect to ATP) is added at a flow and concentration such as to produce a concentration of 0.5 mM in the final flow. The processing fluid is pH7.4 phosphate buffered saline or TrisHCl pH7.8 including 0.2mM magnesium ions.

### EXAMPLE 3: Tubing and reagent network for apparatus of Example 2.

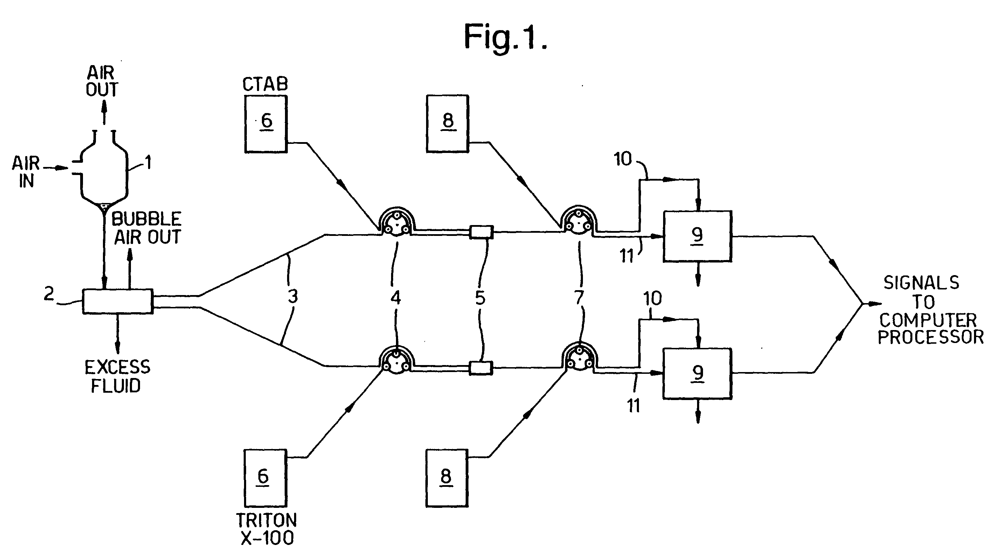

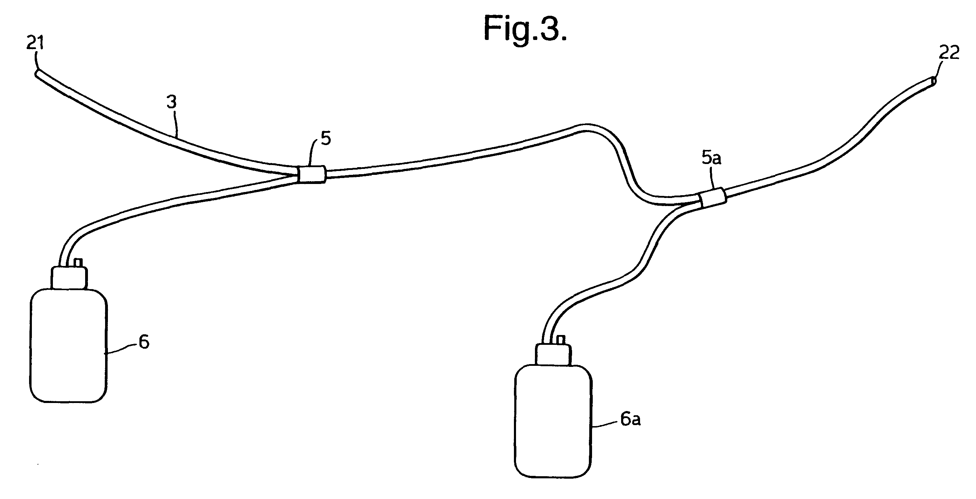

A tubing/reagent replenishment pack provided for the apparatus of Example 2 is shown in Figure 3. All tubing is of silicone rubber of 0.8mm internal diameter joined at durable resilent plastics junctions dimensioned to receive them. The tubing has puncturable sealed ends for connection to one of the interface outputs at a first end (21) and to the luminometer light measuring chamber inlet at the other (22). Container (6) holds detergent reagent and has a sealable air inlet for allowing compensating air to enter as reagent is drawn out under influence of the peristaltic pump (as 4 in Figure 1) on the tubing from the container to junction (5); this pump simultaneously acting upon tubing (3). A further piece of tubing connects junction (5) to junction (5a) where a tube from a second reagent container (6a) carries ADP reagent under influence of a further peristaltic pump (not shown in Figure 1). The tubing between junction (5a) and the end (22) is acted upon by a still further peristaltic pump (as 7 in Figure 1) which also acts upon a tube feeding luminescent reagent to the luminometer chamber.

In preferred apparatus the flow cells are included in the tubing pack and are also disposable; these being made of polycarbonate or polystyrene.

### EXAMPLE 4: Calibration and operation of apparatus of Example 1 or 2.

The amount of ATP present in a typical bacterial cell is of the order of 10⁻¹⁸ moles, equal to about 10⁻¹⁵ grams (see Lundin A (1989) ATP Luminescence- Rapid Methods in Microbiology ed. Stanley P E et al; Blackwell, Oxford ppll-30; and Stanley P E (1989) J. Biolumin. Chemilum, 4, pp375-380). Levels may be expected to be reduced in aerobic organisms when deprived of oxygen and the intracellular concentration may vary typically between 2 and 10mM.

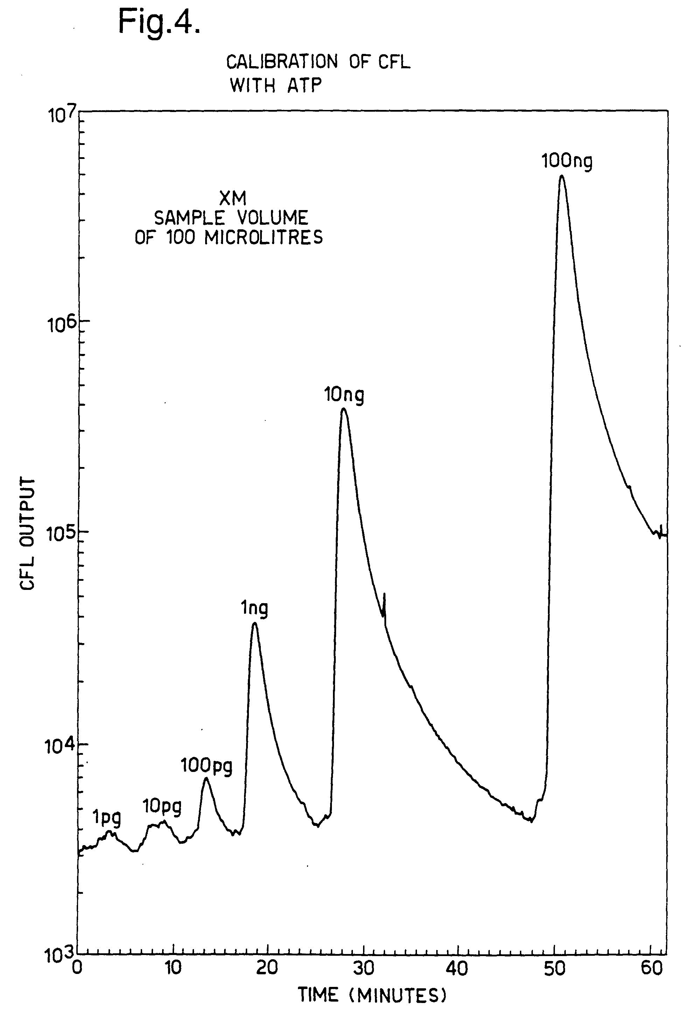

Calibration of the continuous flow luminometers of Example 1 and 2 may be carried out by placing known amounts of ATP into the processing fluid or cyclone while the apparatus is operating and constructing a calibration curve by plotting counts per minute of the luminometer light detector output vs amount of ATP (see Figure 4). Alternatively known concentrations of bacteria such as Bacille Camille Guerre or E. coli may be aerosolised at a set distance, eg. 1 to 10 metres, away from the cyclone inlet and these amounts then plotted against luminometer output as before. Both luminometer light measurement chambers will be exposed to similar amounts of ATP where ATP is used to calibrate, whereas the non-ionic detergent chamber will receive significantly less where bacteria are used; thus a calibration using ATP and bacteria and perhaps eucaryotic cells or fungal spores may be preferable to determine all is operating as it should.

In operation the cyclone supplies particulate sample to the interface from where it is separated into two flows of equal volume and rate by the manifold, each flow containing one of the two detergents, non-ionic or cationic. The flows are mixed with the appropriate amount of ATP/phosphate reagent as required and pass to the luminometer light measurement chambers where luminescence reagents are mixed with them and light emitted. These reagents are preferably of flash kinetic type and allow almost instant emission of light which is detected by light sensors associated with the chambers which in turn pass electrical signals indicative of the amount of ATP detected to a computer processor where the two signals are used to determine the difference in signal, thus ATP, and thus bacterial cells and spores, between the two flows; this being carried out by producing an output to a display unit or printer indicative of raw luminometer output or, using software, a direct estimation of bacteria present as derived by reference to calibration curve stored in a processor associated memory.

The whole operation of the pumps may be controlled by the processor in accordance with preprogrammed regime. Thus if conditions are very dry it might be desired to alter the cyclone rate of air collection or the rate at which sample is passed to or through the interface. Alternatively supplementary collection fluid may be added at the cyclone to dilute the sample if required. Other controls involving rate of operation of one or all of the peristaltic pumps and rate of movement of tape driven immobilised luciferase may also be so controlled as will be appreciated by those skilled in the art.

The preferred apparatus of the invention as shown in Figure 2 uses feedback from the interface to control liquid addition to the cyclone.

## Claims

1. A method for determining the presence and/or amount of cellular material present in a gaseous environment comprising:
(a) continuously collecting a particulate fraction from that environment over a period of time;
(b) continuously transferring the particulate fraction to a stream of processing fluid;
(c) continuously releasing intracellular contents including ATP from microorganisms cells or spores present in the said stream of processing fluid containing the particulate fraction;
(d) continuously adding luminescent reagents dependent upon presence of ATP to the stream of processing fluid to effect luminescence;
(e) measuring light emitted from the stream of processing fluid produced in (d) in a luminometer (9) wherein a signal indicative of this light is produced by the luminometer (9) and the presence and magnitude of the signal is equated to presence and/or amount of cellular material present in the gas.

2. A method as claimed in claim 1 wherein the material comprises bacterial cells or eucaryotic cells.

3. A method as claimed in claim 1 or 2 wherein the gas is atmospheric air.

4. A method as claimed in any one of the preceding claims wherein step (b) is carried out using a lytic agent.

5. A method as claimed in claim 4 wherein the lytic agent is a detergent or an enzyme.

6. A method as claimed in any one of claims 1 to 4 wherein step (b) is carried out using an energy source.

7. A method as claimed in claim 6 wherein the energy source is heat or sound source.

8. A method as claimed in any one of the preceding claims wherein the stream of processing fluid is passed from the collecting step to the other steps via one or more conduits (3).

9. A method as claimed in claim 8 wherein the processing fluid is a liquid.

10. A method as claimed in claim 8 or 9 wherein the fluid is passed along the conduit or conduits (3) by means of pumps (6, 8).

11. A method as claimed in claim 10 wherein the pumps (6,8) are peristaltic pumps and the conduit (3) comprises peristaltic tubing on which the pumps act.

12. A method as claimed in any one of the preceding claims wherein the step (b) produces processing fluid in two separate streams and the step (c) is carried out by use of different means in each flow; whereby in a first one of the flows all cellular material has its intracellular contents released and in the second one of the flows eucaryotic cells and fungal spores have their intracellular contents released; the signal generated from the luminometer light detector (9) in the second flow is subtracted from that from the first and related to the number of bacteria present in the gas taken into the collection step.

13. A method as claimed in claim 12 wherein the first one of the flows is treated with cationic detergent and the second one of the flows is treated with non-ionic detergent.

14. A method as claimed in any one of the preceding claims wherein adenosine diphosphate (ADP) is added to the processing fluid such as to be converted by any adenylate kinase present in the intracellular contents released in step (c) into adenosine triphosphate which in turn is detected in step (e).

15. A method as claimed in any one of the preceding claims wherein a sterile or clean space is being monitored wherein the method continuously collects particulates from the air while various vents supplying air conditioned air are operated or closed down, and ATP content or adenylate kinase content of a batch or sample of processing fluid corresponding to the period of operation is measured using the on-line ATP measuring capability of the method of the invention.

16. An apparatus comprising
(a) a means (1) for continuously collecting a particulate fraction from a gaseous environment;
(b) a means (2) for continuously transferring the particulate fraction to a stream of a processing fluid;
(c) a means (5,6) for continuously releasing intracellular contents including ATP from cellular material present in the stream of processing fluid;
(d) a means (7,8) for continuously adding luminescent reagents, dependent upon presence of ATP to effect luminescence, to the stream of processing fluid;
(e) a light detector means (9) adapted to be fed with the processing fluid from step (d) and capable of emitting a signal indicative of the occurrence and amount of luminescence detected thereby; and
(g) a signal transmitting means for feeding the signal from the luminometer to a processor and/or display means for indicating the presence and/or amount of microorganism cells or spores.

17. An apparatus as claimed in claim 16 wherein the continuous collecting means (1) is a cyclone or a virtual impactor.

18. An apparatus as claimed in claim 17 wherein the collecting means is a cyclone (1) capable of processing over 100 litres of air per minute.

19. An apparatus as claimed in claim 18 wherein the cyclone (1) is capable of processing between 500 and 2000 litres of air per minute.

20. An apparatus as claimed in claim 19 wherein the cyclone (1) processes about 1000 litres of air per minute.

21. An apparatus as claimed in any one of claims 17 to 20 wherein the cyclone (1) is a wet walled hydrocyclone.

22. An apparatus as claimed in any one of claims 16 to 21 wherein the particulate fraction is provided in the form of a liquid processing fluid containing particulate.

23. An apparatus as claimed in claim 17 wherein the collecting means (1) is a high velocity virtual impactor capable of processing between 50 and 150 litres of air per minute.

24. An apparatus as claimed in any one of claims 16 to 23 which further comprises fluid which is a liquid.

25. An apparatus as claimed in claim 24 wherein the liquid is water or a buffer.

26. An apparatus as claimed in claim 25 wherein the water or buffer contains ADP and/or reagent for releasing intracellular contents including ATP from cells.

27. An apparatus as claimed in any one of claims 16 to 26 further comprising a gas liquid interface (2) that is capable of maintaining the dilution of the particulate in the processing fluid at a substantially constant level and/or removing excess air as bubbles.

28. An apparatus as claimed in any one of claims 16 to 27 wherein the means (c) for releasing intracellular content including ATP includes a heater, sonic device or device for adding a lytic agent (6).

29. An apparatus as claimed in any one of claims 16 to 28 wherein the processing fluid is transferred between means in a conduit (3).

30. An apparatus as claimed in claim 29 wherein the fluid is a liquid, the conduit comprises peristaltic tubing and the apparatus includes peristaltic pumps for acting upon this to drive the processing liquid from means to means.

31. An apparatus as claimed in claim 30 wherein the means for releasing intracellular contents and/or supplying ADP comprises a supply of lytic agent and/or ADP reagent which is mixed with the processing liquid at a junction of the peristaltic tubing from the collecting means with tubing from the supply of lytic agent and/or ADP reagent.

32. An apparatus as claimed in claim 31 wherein the junction is at a manifold or at a junction between pieces of peristaltic tubing.

33. An apparatus as claimed in any one of claims 16 to 32 wherein the luminescence reagents are mixed with the processing fluid in the luminometer light measuring device.

34. An apparatus as claimed in any one of claims 16 to 33 wherein the processing fluid is provided as two flows, each flow passing through a respective intracellular contents releasing means capable of releasing ATP or adenylate kinase from either eucaryotic cells and fungal spores or all cellular material, and subsequently passing these flows into respective luminometer light measuring chambers where luminescence reagent adding means provide for emission of light in the presence of ATP; the amount of light detected in the measuring chambers being detected by light detectors which emit electrical signals to a processing or display or print out means.

35. An apparatus as claimed in claim 34 wherein the signal from the eucaryotic cells and fungal spores line is subtracted from the signal from the cellular material line and the value left indicated on a display means or print out means.

36. An apparatus as claimed in any one of claims 16 to 35 wherein the luminescence reagents include luciferase and this is immobilised near the luminometer light detector within the light measuring chamber where the processing fluid and luminescence reagents are mixed.

37. A tubing element or network of such elements suitable for use in the apparatus of the invention comprising a peristaltic tubing element or network of such elements wherein the element or network has a first tubing length with a free end (21) which is suitable for attachment to the fluid outlet of an apparatus for continuously collecting a particulate fraction from a gaseous environment, a further tubing length which has a free end (22) which is suitable for connection to an inlet means of a luminometer chamber (9), other ends of said first and further tubing lengths being interconnected by a tubing length which includes two or more junctions (5, 5a), each junction being connected to a reagent container (6, 6a) by means of a tubing length, characterised in that each reagent container contains a reagent for use in the method of claim 1, the amount and concentration of said reagents being such that the tubing network may be included within an apparatus of any one of claims 16 to 36 and the various reagents in the containers (6,6a) last similar lengths of operation time when the apparatus is used for performing a method as claimed in any one of claims 1 to 15.

38. A tubing element or network as claimed in claim 37 characterised in that the free ends of the tubing are covered to exclude entry of cellular materials by a puncturable or removable end cover.

## Patentansprüche

1. Verfahren zur Ermittlung des Vorliegens und/oder Menge von zellulärem Material in einer gasförmigen Umgebung, das folgende Schritte umfaßt:
(a) kontinuierliches Sammeln einer Partikelfraktion aus dieser Umgebung während einer Zeitdauer,
(b) kontinuierliche Einführung der Partikelfraktion in den Strom eines Verarbeitungsfluids,
(c) kontinuierliche Freisetzung des Zellinhalts einschließlich ATP aus den Zellen oder Sporen von Mikroorganismen, die in dem Strom des Verarbeitungsfluids vorliegen, das die Partikelfraktion enthält,
(d) kontinuierliche Zufuhr von Lumineszenzreagentien in den Strom des Verarbeitungsfluids, um in Abhängigkeit vom Vorliegen von ATP Lumineszenz zu erzeugen,
(e) Messung des vom in (d) erzeugten Strom des Verarbeitungsfluids emittierten Lichts in einem Lumineszenzmeßgerät (9) unter Erzeugung eines diesem Licht entsprechenden Signals und Korrelation des Vorliegens und der Größe des Signals mit dem Vorliegen und/oder der Menge des im Gas vorliegenden zellulären Materials.

2. Verfahren nach Anspruch 1, wobei das zelluläre Material Bakterienzellen und eukaryotische Zellen umfaßt.

3. Verfahren nach Anspruch 1 oder 2, wobei das Gas Atmosphärenluft ist.

4. Verfahren nach einem der vorhergehenden Ansprüche, wobei Schritt (b) unter Verwendung eines Lysemittels durchgeführt wird.

5. Verfahren nach Anspruch 4, wobei das Lysemittel ein Detergens oder ein Enzym ist.

6. Verfahren nach einem der Ansprüche 1 bis 4, wobei Schritt (b) unter Verwendung einer Energiequelle durchgeführt wird.

7. Verfahren nach Anspruch 6, wobei die Energiequelle eine Wärmequelle oder ein Schallquelle ist.

8. Verfahren nach einem der vorhergehenden Ansprüche, wobei der Strom der Verarbeitungsflüssigkeit vom Sammelschritt über eine oder mehrere Leitungen (3) zu den anderen Schritten geleitet wird.

9. Verfahren nach Anspruch 8, wobei das Verarbeitungsfluid eine Flüssigkeit ist.

10. Verfahren nach Anspruch 8 oder 9, wobei das Fluid durch Pumpen (6, 8) über die Leitung oder Leitungen (3) gefördert wird.

11. Verfahren nach Anspruch 10, wobei die Pumpen (6, 8) Peristaltikpumpen sind und die Leitung (3) Peristaltikschläuche umfaßt, auf welche die Pumpen wirken.

12. Verfahren nach einem der vorhergehenden Ansprüche, wobei in Schritt (b) Verarbeitungsfluid in zwei getrennten Strömen erzeugt wird und Schritt (c) unter Verwendung von für jeden Strom verschiedenen Mitteln durchgeführt wird, wobei in einem ersten dieser Ströme der Zellinhalt des gesamten zellulären Materials freigesetzt wird und im zweiten Strom der Zellinhalt von eukaryotischen Zellen und Pilzsporen freigesetzt wird, und das vom Lichtdetektor (9) des Lumineszenzmeßgeräts für den zweiten Strom erzeugte Signal vom für den ersten Strom erhaltenen Signal subtrahiert und mit der Anzahl der Bakterien korreliert wird, die in dem im Sammelschritt gesammelten Gas vorhanden sind.

13. Verfahren nach Anspruch 12, wobei der erste der Ströme mit einem kationischen Detergens und der zweite Strom mit einem nichtionischen Detergens behandelt wird.

14. Verfahren nach einem der vorhergehenden Ansprüche, wobei Adenosindiphosphat (ADP) in der Weise in das Verarbeitungsfluid eingebracht wird, daß es durch eine Adenylatkinase, die in dem in Schritt (c) freigesetzten Zellinhalt vorhanden ist, zu Adenosintriphosphat umgesetzt wird, das seinerseits in Schritt (e) erfaßt wird.

15. Verfahren nach einem der vorhergehenden Ansprüche, wobei ein steriler oder reiner Raum überwacht und partikelförmiges Material kontinuierlich aus der Luft gesammelt wird, wobei verschiedene Zuleitungen zur Zufuhr von klimatisierter Luft geöffnet oder geschlossen werden und der Gehalt einer Charge oder Probe des Verarbeitungsfluids, die der Betriebsdauer entspricht, an ATP oder Adenylatkinase unter Verwendung einer Online-ATP-Meßeinrichtung nach dem erfindungsgemäßen Verfahren gemessen wird.

16. Vorrichtung, die aufweist:
(a) eine Sammeleinrichtung (1) zum kontinuierlichen Sammeln einer Partikelfraktion aus einer gasförmigen Umgebung,
(b) eine Einführungseinrichtung (2) zur kontinuierlichen Einführung der Partikelfraktion in den Strom eines Verarbeitungsfluids,
(c) eine Freisetzungseinrichtung (5, 6) zur kontinuierlichen Freisetzung des Zellinhalts einschließlich ATP aus dem im Strom des Verarbeitungsfluids vorliegenden zellulären Material,
(d) eine Zufuhreinrichtung (7, 8) zur kontinuierlichen Zufuhr von Lumineszenzreagentien in den Strom des Verarbeitungsfluids, um in Abhängigkeit vom Vorliegen von ATP Lumineszenz zu erzeugen,
(e) eine Lichtdetektoreinrichtung (9), die so ausgebildet ist, daß ihr Verarbeitungsfluid von Schritt (d) zugeführt werden kann, und dazu befähigt ist, ein Signal abzugeben, das dem Auftreten und der Intensität der damit erfaßten Lumineszenz entspricht, und
(g) eine Signalübertragungseinrichtung zur Weiterleitung des Signals vom Lumineszenzmeßgerät zu einem Prozessor und/oder einer Anzeigeeinrichtung zur Anzeige des Vorliegens und/oder der Menge von Zellen oder Sporen von Mikroorganismen.

17. Vorrichtung nach Anspruch 16, bei der die kontinuierlich arbeitende Sammeleinrichtung (1) ein Zyklon oder ein virtueller Partikelsammler ist.

18. Vorrichtung nach Anspruch 17, bei der die Sammeleinrichtung ein Zyklon (1) ist, der in der Lage ist, mehr als 100 Liter Luft pro Minute zu verarbeiten.

19. Vorrichtung nach Anspruch 18, wobei der Zyklon (1) in der Lage ist, 500 bis 2000 Liter Luft pro Minute zu verarbeiten.

20. Vorrichtung nach Anspruch 19, wobei der Zyklon (1) etwa 1000 Liter Luft pro Minute verarbeitet.

21. Vorrichtung nach einem der Ansprüche 17 bis 20, bei welcher der Zyklon (1) ein Hydrozyklon mit nassen Wänden ist.

22. Vorrichtung nach einem der Ansprüche 16 bis 21, bei der die Partikelfraktion in Form eines flüssigen Verarbeitungsfluids, das die Partikel enthält, vorliegt.

23. Vorrichtung nach Anspruch 17, bei der die Sammeleinrichtung (1) ein mit hoher Geschwindigkeit arbeitender Partikelsammler ist, der befähigt ist, 50 bis 150 Liter Luft pro Minute zu verarbeiten.

24. Vorrichtung nach einem der Ansprüche 16 bis 23, die ferner ein Fluid aufweist, das eine Flüssigkeit ist.

25. Vorrichtung nach Anspruch 24, bei der die Flüssigkeit Wasser oder eine Pufferlösung ist.

26. Vorrichtung nach Anspruch 25, wobei das Wasser oder die Pufferlösung ADP und/oder ein Reagens zur Freisetzung des Zellinhalts einschließlich ATP aus den Zellen enthält.

27. Vorrichtung nach einem der Ansprüche 16 bis 26, die ferner eine Gas-Flüssig-Grenzfläche (2) aufweist, die in der Lage ist, die Verdünnung des partikelförmigen Materials im Verarbeitungsfluid auf einem im wesentlichen konstanten Niveau zu halten und/oder überschüssige Luft in Form von Blasen abzutrennen.

28. Vorrichtung nach einem der Ansprüche 16 bis 27, bei der die Freisetzungseinrichtung (c) zur Freisetzung des Zellinhalts einschließlich ATP eine Heizeinrichtung, eine Einrichtung zum Beschallen oder eine Einrichtung zur Zuführung eines Lysemittels (6) ist.

29. Vorrichtung nach einem der Ansprüche 16 bis 28, bei der das Verarbeitungsfluid in einer Leitung (3) zwischen den Einrichtungen transportiert wird.

30. Vorrichtung nach Anspruch 29, wobei das Verarbeitungsfluid eine Flüssigkeit ist und die Leitung Peristaltikschläuche umfaßt und die Vorrichtung Peristaltikpumpen aufweist, die auf die Peristaltikschläuche einwirken, um die Verarbeitungsflüssigkeit zwischen den Einrichtungen zu transportieren.

31. Vorrichtung nach Anspruch 30, bei der die Freisetzungseinrichtung zur Freisetzung des Zellinhalts und/oder die Zufuhreinrichtung für ADP eine Einrichtung zur Zufuhr eines Lysemittels und/oder eines ADP-Reagens umfaßt, das an einer Verbindungsstelle der Peristaltikschlauchleitung von der Sammeleinrichtung mit der Schlauchleitung von der Einrichtung zur Zufuhr des Lysemittels und/oder des ADP-Reagens mit der Verarbeitungsflüssigkeit eingemischt wird.

32. Vorrichtung nach Anspruch 31, bei der die Verbindungsstelle an einer Leitungsverzweigung oder einer Verbindung zwischen Teilen der Peristaltikschlauchleitung vorgesehen ist.

33. Vorrichtung nach einem der Ansprüche 16 bis 32, bei der die Lumineszenzreagentien im Lumineszenzmeßgerät mit dem Verarbeitungsfluid gemischt werden.

34. Vorrichtung nach einem der Ansprüche 16 bis 33, bei der das Verarbeitungsfluid in Form von zwei Strömen vorgesehen ist, wobei jeder Strom durch eine entsprechende Freisetzungseinrichtung zur Freisetzung des Zellinhalts hindurchströmt, die in der Lage ist, ATP oder Adenylatkinase aus eukaryotischen Zellen und Pilzsporen bzw. aus dem gesamten zellulären Material freizusetzen, und diese Ströme anschließend in entsprechende Meßkammem eines Lumineszenzmeßgeräts geleitet werden, wo die Zufuhreinrichtung zur Zufuhr von Lumineszenzreagens bei Vorliegen von ATP zur Emission von Licht führt, wobei die Intensität des in den Meßkammem erfaßten Lumineszenzlichts durch Lichtdetektoren erfaßt wird, die elektrische Signale an eine Verarbeitungseinrichtung oder eine Anzeige- oder Ausdruckeinrichtung abgeben.

35. Vorrichtung nach Anspruch 34, bei der das Signal von der Signalleitung für eukaryotische Zellen und Pilzsporen vom Signal von der Signalleitung für das zelluläre Material subtrahiert wird und der erhaltene Differenzwert auf einer Anzeigeeinrichtung angezeigt oder mit einer Ausdruckeinrichtung ausgedruckt wird.

36. Vorrichtung nach einem der Ansprüche 16 bis 35, bei der die Lumineszenzreagentien Luciferase enthalten und diese in der Nähe des Lichtdetektors des Lumineszenzmeßgeräts innerhalb der Meßkammer immobilisiert ist, wo das Verarbeitungsfluid und die Lumineszenzreagentien gemischt werden.

37. Leitungselement oder Leitungsnetzwerk von Leitungselementen, die zur Verwendung in der erfindungsgemäßen Vorrichtung geeignet sind und ein Peristaltikschlauchelement oder ein Netzwerk solcher Elemente umfassen, wobei das Peristaltikschlauchelement oder das Netzwerk aufweisen:
einen ersten Leitungsabschnitt mit einem freien Ende (21), das zum Anschluß am Fluidauslaß einer Vorrichtung zum kontinuierlichen Sammeln einer Partikelfraktion aus einer gasförmigen Umgebung geeignet ist, und einen weiteren Leitungsabschnitt, der ein freies Ende (22) aufweist, das zum Anschluß an die Einlaßeinrichtung einer Meßkammer (9) eines Lumineszenzmeßgeräts geeignet ist, wobei die anderen Enden des ersten und des weiteren Leitungsabschnitts über einen Leitungsabschnitt miteinander verbunden sind, der zwei oder mehr Abzweigungen (5, 5a) aufweist, wobei jede Abzweigung über einen Leitungsabschnitt mit einem Reagensbehälter (6, 6a) verbunden ist, dadurch gekennzeichnet, daß jeder Reagensbehälter ein Reagens zur Verwendung beim Verfahren von Anspruch 1 aufweist, wobei die Menge und Konzentration dieser Reagentien so sind, daß das Leitungsnetzwerk in einer Vorrichtung nach einem der Ansprüche 16 bis 36 vorgegeben werden kann und die verschiedenen Reagentien in den Reagensbehältern (6, 6a) für ähnliche Betriebsdauem reichen, wenn die Vorrichtung zur Durchführung eines Verfahrens nach einem der Ansprüche 1 bis 15 verwendet wird.

38. Leitungselement oder Leitungsnetzwerk nach Anspruch 37, dadurch gekennzeichnet, daß die freien Enden der Leitungen mit einem durchstechbaren oder abnehmbaren Endverschluß verschlossen sind, um einen Eintritt zellulärer Materialien auszuschließen.

## Revendications

1. Procédé pour déterminer la présence et/ou la quantité de matière cellulaire présente dans un environnement gazeux, comportant :
(a) la récupération en continu d'une fraction de particules à partir de cet environnement sur une période de temps,
(b) le transfert en continu de la fraction de particules vers un flux de fluide de traitement,
(c) la libération en continu des contenus intracellulaires, y compris l'ATP à partir de cellules de micro-organismes ou de spores présents dans ledit flux de fluide de traitement contenant la fraction de particules,
(d) l'ajout en continu de réactifs luminescents en fonction de la présence d'ATP, au flux de fluide de traitement pour effectuer une luminescence,
(e) la mesure de la lumière émise à partir du flux de fluide de traitement produit à l'étape (d) dans un luminomètre, un signal représentatif de cette lumière étant produit par le luminomètre et la présence et l'amplitude du signal étant en équation avec la présence et/ou la quantité de matière cellulaire présente dans le gaz.

2. Procédé selon la revendication 1, dans lequel la matière est constituée de cellules bactériennes ou de cellules eucaryotes.

3. Procédé selon la revendication 1 ou 2, dans lequel le gaz est de l'air de l'atmosphère.

4. Procédé selon l'une quelconque des revendications précédentes, dans lequel l'étape (b) est mise en oeuvre en utilisant un agent lytique.

5. Procédé selon la revendication 4, dans lequel l'agent lytique est un détergent ou une enzyme.

6. Procédé selon l'une quelconque des revendications 1 à 4, dans lequel l'étape (b) est mise en oeuvre en utilisant une source d'énergie.

7. Procédé selon la revendication 6, dans lequel la source d'énergie est une source de chaleur ou une source sonore.

8. Procédé selon l'une quelconque des revendications précédentes, dans lequel le flux de fluide de traitement est envoyé depuis l'étape de récupération vers les autres étapes via un ou plusieurs conduits (3).

9. Procédé selon la revendication 8, dans lequel le fluide de traitement est un liquide.

10. Procédé selon la revendication 8 ou 9, dans lequel le liquide est envoyé le long du conduit ou des conduits (3) par l'intermédiaire de pompes (6, 8).

11. Procédé selon la revendication 10, dans lequel les pompes (6, 8) sont des pompes péristaltiques et le conduit (3) est constitué d'une tuyauterie péristaltique sur laquelle agissent les pompes.

12. Procédé selon l'une quelconque des revendications précédentes, dans lequel l'étape (b) produit un fluide de traitement dans deux flux séparés et l'étape (c) est mise en oeuvre en utilisant des moyens différents dans chaque écoulement, de sorte que dans un premier des écoulements, toute la matière cellulaire a son contenu intracellulaire libéré et dans le second des écoulements, les cellules eucaryotes et les spores fongiques ont leurs contenus intracellulaires libérés, le signal produit par le détecteur de lumière du luminomètre (9) situé dans le second écoulement est soustrait de celui du premier et est fonction du nombre de bactéries présentes dans le gaz pris à l'étape de récupération.

13. Procédé selon la revendication 12, dans lequel le premier des écoulements est traité à l'aide d'un détergent cationique et le second des écoulements est traité à l'aide d'un détergent non-ionique.

14. Procédé selon l'une quelconque des revendications précédentes, dans lequel l'adénosine-diphosphate (ADP) est ajouté au fluide de traitement de manière à être converti par toute adénylate-kinase présente dans le contenu intracellulaire libéré à l'étape (c) en adénosine-triphosphate qui à son tour est détecté à l'étape (e).

15. Procédé selon l'une quelconque des revendications précédentes, dans lequel un espace stérile ou propre est surveillé dans lequel le procédé récupère en continu des particules à partir de l'air alors que diverses ouvertures alimentant de l'air conditionné en air sont activées ou fermées, et la teneur en ATP ou la teneur en adénylate-kinase d'un lot ou d'un échantillon de fluide de traitement correspondant à la période de fonctionnement est mesurée en utilisant la capacité de mesure d'ATP en ligne du procédé de la présente invention.

16. Dispositif comportant :
(a) des moyens (1) pour récupérer en continu une fraction de particules à partir d'un environnement gazeux,
(b) des moyens (2) pour transférer en continu la fraction de particules vers un flux de fluide de traitement,
(c) des moyens (5, 6) pour libérer en continu les contenus intracellulaires y compris l'ATP à partir de la matière cellulaire présente dans le flux de fluide de traitement,
(d) des moyens (7, 8) pour ajouter en continu des réactifs luminescents, en fonction de la présence d'ATP pour effectuer une luminescence, au flux de fluide de traitement,
(e) des moyens détecteurs de lumière (9) adaptés pour être alimentés en fluide de traitement provenant de l'étape (d) et pouvant émettre un signal représentatif de l'existence et de la quantité de luminescence détectée de la sorte, et
(g) des moyens de transmission de signal pour acheminer le signal provenant du luminomètre vers un processeur et/ou des moyens d'affichage pour indiquer la présence et/ou la quantité de cellules de micro-organismes ou de spores.

17. Dispositif selon la revendication 16, dans lequel les moyens de récupération en continu (1) sont un cyclone ou un dispositif à impact efficace.

18. Dispositif selon la revendication 17, dans lequel les moyens de récupération sont un cyclone (1) pouvant traiter plus de 100 litres par minute.

19. Dispositif selon la revendication 18, dans lequel le cyclone (1) est capable de traiter entre 500 et 2000 litres d'air par minute.

20. Dispositif selon la revendication 19, dans lequel le cyclone (1) traite environ 1000 litres d'air par minute.

21. Dispositif selon l'une quelconque des revendications 17 à 20, dans lequel le cyclone (1) est un hydrocyclone à paroi humide.

22. Dispositif selon l'une quelconque des revendications 16 à 21, dans lequel la fraction de particules est fournie sous la forme d'un fluide de traitement liquide contenant des particules.

23. Dispositif selon la revendication 17, dans lequel les moyens de récupération (1) sont un dispositif à impact efficace à vitesse élevée capable de traiter entre 50 et 150 litres d'air par minute.

24. Dispositif selon l'une quelconque des revendications 16 à 23, qui comporte en outre un fluide qui est un liquide.

25. Dispositif selon la revendication 24, dans lequel le liquide est de l'eau ou un tampon.

26. Dispositif selon la revendication 25, dans lequel l'eau ou le tampon contient de l'ADP et/ou un réactif destiné à libérer le contenu intracellulaire y compris ATP à partir des cellules.

27. Dispositif selon l'une quelconque des revendications 16 à 26, comportant de plus une interface gaz-liquide (2) qui est capable de maintenir la dilution des particules dans le fluide de traitement à un niveau sensiblement constant et/ou d'enlever l'air en excès sous forme de bulles.

28. Dispositif selon l'une quelconque des revendications 16 à 27, dans lequel les moyens (c) pour libérer le contenu cellulaire y compris l'ATP comportent un dispositif de chauffage, un dispositif sonore ou un dispositif destiné à ajouter un agent lytique (6).

29. Dispositif selon l'une quelconque des revendications 16 à 28, dans lequel le fluide de traitement est transféré entre des moyens dans un conduit (3).

30. Dispositif selon la revendication 29, dans lequel le fluide est un liquide, le conduit est constitué d'une tuyauterie péristaltique et le dispositif inclut des pompes péristaltiques destinées à agir sur celle-ci pour entraîner le fluide de traitement depuis des moyens vers d'autres moyens.

31. Dispositif selon la revendication 30, dans lequel les moyens pour libérer le contenu intracellulaire et/ou alimenter de l'ADP comportent une alimentation en agent lytique et/ou réactif d'ADP, qui est mélangé avec le liquide de traitement au niveau d'une jonction de la tuyauterie péristaltique provenant des moyens de récupération avec la tuyauterie provenant de l'alimentation en agent lytique et/ou réactif d'ADP.

32. Dispositif selon la revendication 31, dans lequel la jonction est située au niveau d'un collecteur ou au niveau d'une jonction entre des parties de la tuyauterie péristaltique.

33. Dispositif selon l'une quelconque des revendications 16 à 32, dans lequel les réactifs de luminescence sont mélangés avec le fluide de traitement dans le dispositif de mesure de lumière du luminomètre.

34. Dispositif selon l'une quelconque des revendications 16 à 33, dans lequel le fluide de traitement est fourni sous la forme de deux écoulements, chaque écoulement passant à travers des moyens de libération de contenu intracellulaire respectif capables de libérer l'ATP ou l'adénylate-kinase à partir de cellules eucaryotes et de spores fongiques ou de toute matière cellulaire, et faisant passer par la suite ces écoulements dans des chambres respectives de mesure de lumière de luminomètre où les moyens d'ajout de réactif de luminescence assurent une émission de lumière en présence d'ATP, la quantité de lumière détectée dans les chambres de mesure étant détectée par des détecteurs de lumière qui émettent des signaux électriques vers des moyens de traitement ou d'affichage ou d'impression.

35. Dispositif selon la revendication 34, dans lequel le signal provenant de la ligne de cellules eucaryotes et de spores fongiques est soustrait du signal provenant de la ligne de matière cellulaire et la valeur obtenue est indiquée sur des moyens d'affichage ou d'impression.

36. Dispositif selon l'une quelconque des revendications 16 à 35 dans lequel les réactifs de luminescence comportent la luciférase et celle-ci est immobilisée à proximité du détecteur de lumière du luminomètre dans la chambre de mesure de lumière où le fluide de traitement et les réactifs de luminescence sont mélangés.

37. Elément de tuyauterie ou réseau de tuyauterie constitué d'éléments adaptés pour être utilisés dans le dispositif de la présente invention, comportant un élément ou un réseau de tuyauterie péristaltique constitué de tels éléments dans lesquels l'élément ou le réseau a une première longueur de tuyauterie ayant une extrémité libre (21) qui est adaptée pour être fixée à la sortie de fluide d'un dispositif de récupération en continu d'une fraction de particules à partir d'un environnement gazeux, une autre longueur de tuyauterie qui a une extrémité libre (22) qui est adaptée pour être reliée à des moyens d'entrée d'une chambre de luminomètre (9), d'autres extrémités de ladite première et d'autres longueurs de tuyauterie étant reliées par une longueur de tuyauterie qui comporte deux ou plus de deux jonctions (5, 5a), chaque jonction étant reliée à un conteneur de réactif (6, 6a) par l'intermédiaire d'une longueur de tuyauterie, caractérisé en ce que chaque conteneur de réactif contient un réactif destiné à être utilisé dans le procédé de la revendication 1, la quantité et la concentration desdits réactifs étant telles que le réseau de tuyauterie peut être inclus dans un dispositif selon l'une quelconque des revendications 16 à 36 et les divers réactifs situés dans les conteneurs (6, 6a) ont une durée similaire aux longueurs de temps de fonctionnement lorsque le dispositif est utilisé pour mettre en oeuvre un procédé selon l'une quelconque des revendications 1 à 15.

38. Elément ou réseau de tuyauterie selon la revendication 37, caractérisé en ce que les extrémités libres de la tuyauterie sont recouvertes pour empêcher l'entrée de matière cellulaire, par un couvercle d'extrémité pouvant être percé ou enlevé.
